# EUROPEAN PATENT SPECIFICATION

(11) **EP 0 613 371 B1**
(45) Date of publication and mention of the grant of the patent: **06.03.2002**
(21) Application number: 92924683.3
(22) Date of filing: 07.12.1992
(51) Int. Cl.: A61K 31/57, A61P 11/08

(54) **NEW COMBINATION OF FORMOTEROL AND BUDESONIDE**
FORMOTEROL UND BUDESONIDE ENTHALTENDE ZUSAMMENSETZUNG
NOUVELLE COMBINAISON DE FORMOTEROL ET DE BUDESONIDE

(30) Priority: 18.12.1991 EP 91311761
(43) Date of publication of application: 07.09.1994
(60) Divisional application: 00127680.7
(73) Proprietor: AstraZeneca AB, 151 85 Södertälje (SE)
(72) Inventor: CARLING, Christer Carl Gustav, S-240 10 Dalby (SE); TROFAST, Jan William, S-226 47 Lund (SE)
(74) Representative: Linderoth, Margareta
(86) International application number: EP9202826
(87) International publication number: WO9311773

(56) References cited:
- ANNALS OF ALLERGY vol. 63, no. 3, September 1989, pages 220 - 224 JEAN H. MARSAC ET AL. 'Inhaled beta agonists and inhaled steroids in the treatment of asthma' cited in the application
- LUNG (USA) vol. 168, no. SUPP, 1990, NEW YORK pages 105 - 110 NILS SVEDMYR 'The current place of beta-agonists in the management of asthma' cited in the application

## Description

### Field of the invention

This invention relates to improvements in the treatment of mild as well as severe asthma and other respiratory disorders. More particularly, it relates to the use of a bronchodilator in combination with a steroidal antiinflammatory drug for the treatment of respiratory disorders such as asthma, and to pharmaceutical compositions containing the two active ingredients. It emphasizes the use of a long-acting bronchodilator which provides rapid relief of symptoms.

### Background of the invention

There have recently been significant advances in our understanding of asthma. Despite many advances, both in awareness of the disease by doctors and patients alike, coupled with the introdction of very powerful and effective anti-asthma drugs, asthma remains a poorly understood and often poorly treated disease. Previously, contraction of airway smooth muscles has been regarded as the most important feature of asthma. Recently there has been a marked change in the way asthma is managed, stemming from the fact that asthma is recognized as a chronic inflammatory disease. Uncontrolled airway inflammation may lead to mucosal damage and structural changes giving irrversible narrowing of the airways and fibrosis of the lung tissue. Therapy should therefore be aimed at controlling symptoms so that normal life is possible and at the same time provide basis for treating the underlying inflammation.

The most common cause for poor control of asthma is poor compliance with the long-term management of chronic asthma, particularly with prophylatic treatments, such as inhaled steroids, which do not give immediate symptom relief. Patients will readily take β₂-agonist inhalers, since these provide rapid relief of symptoms, but often do not take prophylactic therapy, such as inhaled steroids, regularly because there is no immediate symptomatic benefit. They also counteract down regulation of β₂-adrenoceptor agonists.

Formoterol, (N-[2-hydroxy-5-[1-hydroxy-2-[[2-(4-methoxyphenyl)-1-methylethyl]amino]ethyl]phenyl] formamide), is an adrenoceptor agonist which selectively stimulates β₂-receptors, thus producing relaxation of bronchial smooth muscle, inhibition of the release of endogenous spasmogens, inhibition of oedema caused by endogenous mediators, and increased mucociliary clearence. Inhaled formoterol fumarate acts rapidly, usually within minutes which gives the patient immediate confirmation that he has taken an adequat dose and thereby avoiding overdosing of both β-agonist and steroid. Inhaled formoterol also exerts a prolonged bronchodilation, which in clinical trials has been demonstrated as up to 12 hours.

Budesonide, (16,17-butylidenebis(oxy)-11,21-dihydroxypregna-1,4-diene-3,20-dione), may be given in a high inhaled dose (up to 2 mg daily) with very low systemic effects, possibly because of its rapid metabolism. The high rapid systemic elimination of budesonide is due to extensive and rapid hepatic metabolism. Long term clinical studies have shown that inhaled budesonide is a pharmacologically safe drug. High doses of inhaled budesonide are highly effective and well tolerated when used in oral steroid replacement therapy. Budesonide represents a logical safe and effective therapy for long term control of asthma.

The inhaled route of administration enables the dose to be delivered directly to the airways. By this type of administration, it is possible to give a small dose and thereby minimizing unwanted side-effects. The drawbacks of the currently available bronchodilators are their relatively short duration of action. By using a compound with long duration e.g. formoterol it would be possible to avoid the nocturnal asthma, which so often causes considerable anxiety and debility to the patients. Formoterol gives less nocturnal waking than the commonly used short-acting agonists like salbutamol, terbutaline and the like. Formoterol has been registered for oral administration in Japan since 1986.

Pharmaceutical combinations of long-acting β₂-agonists and steroids are disclosed in two European applications, EP 416950 which discloses the combination of salmeterol and beclomethasone, and EP 416951 which discloses the combination of salmeterol and fluticasone propionate.

In Ann. Allergy 1989, 63 (3), p. 220-224 the use of a β₂-agonist, i.e. formoterol and a steroid, i.e. budesonide seperately are mentioned. It is not disclosed a pharmaceutical combination including both formoterol and budesonide, or the use of the two compounds in combination therapy. The use of a β₂-agonist and a steroid separately is also mentoined in Lung (1990), 168, no. supp, p. 105-110.

### Outline of the Invention

The present invention is based on the concept of a novel combination therapy whereby formoterol (and/or a physiologically acceptable salt and/or solvate thereof) and budesonide are administrated simultaneously, sequentially or separately by inhalation. This combination has not only a greater efficiency and duration of bronchodilator action but the combination also has a rapid onset of action. This new feature is of utmost importance in order to establish a higher compliance for patients and it provides a rescue medicine thereby avoiding the necessity for the patient of carrying two different inhalers. This simplifies life for patients considerably and makes life more comfortable and secure. The rapid onset of the long-acting β₂-agonist gives the patient immediate confirmation that he has taken an adequate dose and thereby avoiding overdosing of both β₂-agonist and steroid. Since the use of formoterol instead of salmeterol gives a much more rapid onset the combinations according to the invention have a number of advantages compared to the combinations disclosed in EP 416 950 and EP 416 951. The combination according to present invention permits a twice daily dosing regime as a basic treatment of asthma, particularly nocturnal asthma.

According to one aspect of the invention there is provided a medical product comprising, together,
(i) formoterol or a physiologically acceptable salt thereof, or a solvate of said salt, or a solvate of formoterol; and
(ii) budesonide as a combined preparation for administration by inhalation.

According to a second aspect of the invention there is provided a pharmaceutical composition for administration by inhalation, which comprises together,
(i) formoterol or a physiologically acceptable salt thereof, or a solvate of said salt, or a solvate of formoterol; and
(ii) budesonide.

According to a further aspect according to the invention there is provided use of
(i) formoterol, or a physiologically acceptable salt thereof, or a solvate of said salt, or a solvate of formoterol; and
(ii) budesonide, in the manufacture of a combined preparation for administration by inhalation in the treatment of respiratory disorder.

Further the invention provides the use of formoterol or a physiologically acceptable salt thereof or a solvate of the salt in the manufacture of a medical product according to the invention for administration by inhalation in the treatment of a respiratory disorder and the use of budesonide in the manufacture of a medical product according to the invention for administration by inhalation in the treatment of respiratory disorder.

Suitable physiologically salts of formoterol include acid addition salts derived from inorganic and organic acids, such as the hydrochloride, hydrobromide, sulphate, phosphate, maleate, fumarate, tartrate, citrate, benzoate, 4-methoxybenzoate, 2- or 4-hydroxybenzoate, 4-chlorobenzoate, p-toluenesulphonate, methanesulphonate, ascorbate, salicylate, acetate succinate, lactate, glutarate, gluconate, tricarballylate, hydroxynaphthalenecarboxylate or oleate. Formoterol is preferably used in the form of its fumarate salt and as a dihydrate.

The ratio of formoterol to budesonide used according to the invention is preferably within the range of 1:4 to 1:70. The two drugs may be administered separately in the same ratio.

The intended dose regimen is a twice daily administration, where the suitable daily dose of formoterol is in the range of 6 to 100 µg with a preferred dose of 6-48 µg and the suitable daily dose for budesonide is 50 to 4800 µg with a preferred dose of 100-1600 µg. The particular dose used will strongly depend on the patient (age, weight etc) and the severity of the disease (mild, moderate, severe asthma etc).

For administration, the combination is suitably inhaled from a nebulizer, from a pressurized metered dose inhaler or as a dry powder from a dry powder inhaler (e.g. as sold under the trade mark Turbuhaler) or from a dry powder inhaler utilizing gelatine, plastic or other capsules, cartridges or blister packs.

A diluent or carrier, generally non-toxic and chemically inert to the medicament e.g. lactose, dextran, mannitol or glucose or any additives that will give the medicament a desired taste, can be added to the powdered medicament.

Examples of the preparation of suitable dosage forms according to the invention include the following: Formoterol fumarate dihydrate and budesonide (optionally premicronized) are mixed in the proportions given above. The agglomerated, free-flowing micronized mixture may be filled into dry powder inhaler such as sold under the trade mark Turbuhaler. When a capsule system issued, it is desirable to include a filler in the mixture.

The micronized mixture may be suspended or dissolved in a liquid propellant mixture which is kept in a container that is sealed with a metering valve and fitted into a plastic actuator. The propellants used may be chlorofluorocarbons of different chemical formulae. The most frequently used chlorofluorocarbon propellants are trichloromonofluoromethane (propellant 11), dichlorodifluoromethane (propellant 12), dichlorotetrafluoroethane (propellant 114), tetrafluoroethane (propellant 134a) and 1,1-difuoroethane (propellant 152a). Low concentrations of a surfactant such as sorbitan trioleate, lecithin, disodium dioctylsulphosuccinate or oleic acid may also be used to improve the physical stability.

The invention is further illustrated by way of example with reference to the following Examples.

### Example 1 - Dry Powder Inhaler (Turbuhaler)

| Active ingredient | Per dose |
|---|---|
| Formoterol (as fumarate dihydrate) | 12 µg |
| Budesonide | 200 µg |

The storage unit of the inhaler is filled with sufficient for at least 200 doses.

| Active ingredient | Per dose |
|---|---|
| Formoterol (as fumarate dihydrate) | 24 µg |
| Budesonide | 200 µg |

The storage unit is filled with sufficient for at least 200 doses.

| Active ingredient | Per dose |
|---|---|
| Formoterol (as fumarate dihydrate) | 12 µg |
| Budesonide | 100 µg |

The storage unit is filled with sufficient for at least 200 doses.

### Example 2 - Metered dose inhaler

| Active ingredient | Per dose |
|---|---|
| Formoterol (as fumarate dihydrate) | 12 µg |
| Budesonide | 200 µg |
| Stabilizer | 0.1 - 0.7 mg |
| Propellant | 25 - 100 µl |

| Active ingredient | Per dose |
|---|---|
| Formoterol (as fumarate dihydrate) | 24 µg |
| Budesonide | 200 µg |
| Stabilizer | 0.1 - 0.7 mg |
| Propellant | 25 - 100 µl |

| Active ingredient | Per dose |
|---|---|
| Formoterol (as fumarate dihydrate) | 12 µg |
| Budesonide | 200 µg |
| Stabilizer | 0.1 - 0.7 mg |
| Propellant | 25 - 100 µl |

### Example 3 - Metered dose dry powder formulation

| Active ingredient | Per dose |
|---|---|
| Formoterol (as fumarate dihydrate) | 12 µg |
| Budesonide | 200 µg |
| Lactose | up to 5, 12.5 or 25 mg |

| Active ingredient | Per dose |
|---|---|
| Formoterol (as fumarate dihydrate) | 24 µg |
| Budesonide | 200 µg |
| Lactose | up to 5, 12.5 or 25 mg |

| Active ingredient | Per dose |
|---|---|
| Formoterol (as fumarate dihydrate) | 12 µg |
| Budesonide | 100 µg |
| Lactose | up to 5, 12.5 or 25 mg |

## Claims

1. A medical product comprising, together,
(i) formoterol or a physiologically acceptable salt thereof, or a solvate of said salt, or a solvate of formoterol; and
(ii) budesonide as a combined preparation for administration by inhalation.

2. A medical product according to claim 1, which comprises, as component (i), formoterol fumarate dihydrate.

3. A medical product according to claim 1 or 2, which comprises a non-toxic diluent or carrier.

4. A medical product according to claim 3, in which the non-toxic diluent or carrier is lactose.

5. A medical product according to any of the preceding claims, in which components (i) and (ii) are in dry powder form.

6. A medical product according to any of the preceding claims, in which the ratio of the component (i) to component (ii) is in the range 1:4 to 1: 70.

7. A medical product according to any of the preceding claims, which contains component (i) and (ii) in unit dose form.

8. A medical product according to any of the preceding claims in which components (i) and (ii) are for administration by inhalation in the treatment of a respiratory disorder.

9. A medical product according to any of claims 1 to 8 in which components (i) and (ii) are for administration by inhalation in the treatment of asthma.

10. A medical product according to any of the preceding claims, in which the administration is by inhalation from a dry powder inhaler.

11. A medical product according to any of claims 1 to 9 in which the administration is by inhalation from a pressurized metered dose inhaler.

12. A medical product according to any of claims 1 to 9 in which the administration is by inhalation from a nebulizer.

13. A pharmaceutical composition for administration by inhalation, which comprises, together
(i) formoterol or a physiologically acceptable salt thereof, or a solvate of the salt, or a solvate of formoterol; and
(ii) budesonide.

14. A composition according to claim 13 which comprises, as component (i), formoterol fumarate dihydrate.

15. A composition according to claim 13 or 14 which comprises a non-toxic diluent or carrier.

16. A composition according to claim 15 in which the non-toxic diluent or carrier is lactose.

17. A composition according to any of claims 13 to 16 in which the ratio of the component (I) to component (ii) is in the range 1:4 to 1:70.

18. A composition according to any of claims 13 to 17 in unit dose form.

19. A composition according to any of claims 13 to 18 in which components (i) and (ii) are each in micronised form.

20. A composition according to claim 19 which is in agglomerated form.

21. A dry powder inhaler which contains a composition as claimed in claim 20.

22. A capsule, cartridge or blister pack for a dry powder inhaler, the capsule, cartridge or blister pack containing a composition as claimed in claim 20.

23. A composition as claimed in any one of claims 13 to 20 for use in the treatment by inhalation of respiratory disorder.

24. A composition as claimed in any one of claims 13 to 20 for use in the treatment by inhalation of asthma.

25. Use of
(i) formoterol, or a physiologically acceptable salt thereof, or a solvate of said salt, or a solvate of formoterol; and
(ii) budesonide,
in the manufacture of a combined preparation for administration by inhalation in the treatment of respiratory disorder.

26. Use according to claim 25, in which component (i) is formoterol fumarate dihydrate.

27. Use according to claim 25 or 26 in which the ratio of the component (i) to component (ii) is in the range 1:4 to 1:70.

28. Use according to any of claims 25 to 27 in which the dose regime is twice daily and the dose of component (i) is 6 - 100 µg per day and the dose of component (ii) is 50 - 4800 µg per day.

29. Use according to claim 28 in which the dose of component (i) is 6 - 48 µg per day and the dose of component (ii) is 100 to 1600 µg per day.

30. Use of formoterol, or a physiologically acceptable salt thereof, or a solvate of said salt, or a solvate of formoterol, in the manufacture of a medical product as defined in claim 1, for administration by inhalation in the treatment of a respiratory disorder.

31. Use of budesonide in the manufacture of a medical product as defined in claim 1, for administration by inhalation in the treatment of respiratory disorder.

## Patentansprüche

1. Medizinisches Produkt, enthaltend
(i) Formoterol oder ein physiologisch unbedenkliches Salz davon, oder ein Solvat des Salzes, oder ein Solvat von Formoterol, zusammen mit
(ii) Budesonid, als Kombinationspräparat zur inhalativen Verabreichung.

2. Medizinisches Produkt nach Anspruch 1, welches als Komponente (i) Formoterolfumarat-dihydrat enthält.

3. Medizinisches Produkt nach Anspruch 1 oder 2, welches ein nichttoxisches Verdünnungsmittel oder einen nichttoxischen Trägerstoff enthält.

4. Medizinisches Produkt nach Anspruch 3, bei dem es sich bei dem nichttoxischen Verdünnungsmittel bzw. dem nichttoxischen Trägerstoff um Laktose handelt.

5. Medizinisches Produkt nach einem der vorhergehenden Ansprüche, bei dem die Komponenten (i) und (ii) in Trockenpulverform vorliegen.

6. Medizinisches Produkt nach einem der vorhergehenden Ansprüche, bei dem das Verhältnis von Komponente (i) zu Komponente (ii) im Bereich von 1:4 bis 1:70 liegt.

7. Medizinisches Produkt nach einem der vorhergehenden Ansprüche, welches die Komponenten (i) und (ii) in Einzeldosisform enthält.

8. Medizinisches Produkt nach einem der vorhergehenden Ansprüche, bei dem die Komponenten (i) und (ii) für die inhalative Verabreichung bei der Behandlung einer Atemwegserkrankung bestimmt sind.

9. Medizinisches Produkt nach einem der Ansprüche 1 bis 8, bei dem die Komponenten (i) und (ii) für die inhalative Verabreichung bei der Behandlung von Asthma bestimmt sind.

10. Medizinisches Produkt nach einem der vorhergehenden Ansprüche, bei dem die Verabreichung durch Inhalation aus einem Trockenpulverinhalationsgerät erfolgt.

11. Medizinisches Produkt nach einem der Ansprüche 1 bis 9, in welchem die Verabreichung aus einem unter Druck stehenden Inhalationsgerät zur dosierten Verabreichung erfolgt.

12. Medizinisches Produkt nach einem der Ansprüche 1 bis 9, bei welchem die Verabreichung durch Inhalation aus einem Vernebler erfolgt.

13. Pharmazeutische Zusammensetzung zur inhalativen Verabreichung, enthaltend
(i) Formoterol oder ein physiologisch unbedenkliches Salz davon, oder ein Solvat des Salzes, oder ein Solvat von Formoterol, zusammen mit
(ii) Budesonid.

14. Zusammensetzung nach Anspruch 13, welche als Komponente (i) Formoterolfumarat-dihydrat enthält.

15. Zusammensetzung nach Anspruch 13 oder 14, welche ein nichttoxisches Verdünnungsmittel oder einen nichttoxischen Trägerstoff enthält.

16. Zusammensetzung nach Anspruch 15, bei der es sich bei dem nichttoxischen Verdünnungsmittel bzw. dem nichttoxischen Trägerstoff um Laktose handelt.

17. Zusammensetzung nach einem der Ansprüche 13 bis 16, bei der das Verhältnis von Komponente (i) zu Komponente (ii) im Bereich von 1:4 bis 1:70 liegt.

18. Zusammensetzung nach einem der Ansprüche 13 bis 17 in Einzeldosisform.

19. Zusammensetzung nach einem der Ansprüche 13 bis 18, bei der die Komponenten (i) und (ii) jeweils in mikronisierter Form vorliegen.

20. Zusammensetzung nach Anspruch 19, wobei die Zusammensetzung in agglomerisierter Form vorliegt.

21. Trockenpulverinhalationsgerät, welches eine Zusammensetzung nach Anspruch 20 enthält.

22. Kapsel, Patrone oder Durchdrückpackung für ein Trockenpulverinhalationsgerät, wobei die Kapsel, die Patrone bzw. die Durchdrückpackung eine Zusammensetzung nach Anspruch 20 enthält.

23. Zusammensetzung nach einem der Ansprüche 13 bis 20 zur Verwendung bei der inhalativen Behandlung von Atemwegserkrankungen.

24. Zusammensetzung nach einem der Ansprüche 13 bis 20 zur Verwendung bei der inhalativen Behandlung von Asthma.

25. Verwendung von
(i) Formoterol, oder eines physiologisch unbedenkliches Salzes davon, oder eines Solvates des Salzes, oder eines Solvates von Formoterol, und
(ii) Budesonid
bei der Herstellung eines Kombinationspräparats zur inhalativen Verabreichung bei der Behandlung von Atemwegserkrankungen.

26. Verwendung nach Anspruch 25, wobei es sich bei Komponente (i) um Formoterolfumaratdihydrat handelt.

27. Verwendung nach Anspruch 25 oder 26, wobei das Verhältnis von Komponente (i) zu Komponente (ii) im Bereich von 1:4 bis 1:70 liegt.

28. Verwendung nach einem der Ansprüche 25 bis 27, wobei die Verabreichung zweimal täglich erfolgt und die Dosis von Komponente (i) 6-100 µg pro Tag und die Dosis von Komponente (ii) 50-4800 µg pro Tag beträgt.

29. Verwendung nach Anspruch 28, wobei die Dosis von Komponente (i) 6-48 µg pro Tag und die Dosis von Komponente (ii) 100-1600 µg pro Tag beträgt.

30. Verwendung von Formoterol, oder eines physiologisch unbedenklichen Salzes davon, oder eines Solvates des Salzes, oder eines Solvates von Formoterol, bei der Herstellung eines medizinischen Produktes nach Anspruch 1 für die inhalative Verabreichung bei der Behandlung von Atemwegserkrankungen.

31. Verwendung von Budesonid bei der Herstellung eines medizinischen Produktes nach Anspruch 1 für die inhalative Verabreichung bei der Behandlung von Atemwegserkrankungen.

## Revendications

1. Médicament comprenant conjointement
(i) le formotérol ou un sel physiologiquement acceptable de celui-ci, ou un solvate dudit sel, ou un solvate de formotérol ; et
(ii) le budésonide comme une préparation combinée destinée à l'administration par inhalation.

2. Médicament selon la revendication 1, **caractérisé en ce qu'**il comprend, à titre de composant (i), le fumarate de formotérol dihydraté.

3. Médicament selon la revendication 1 ou 2, **caractérisé en ce qu'**il comprend un diluant ou un support non toxique.

4. Médicament selon la revendication 3, **caractérisé en ce que** le diluant ou le support non toxique est le lactose.

5. Médicament selon l'une quelconque des revendications précédentes, **caractérisé en ce que** les composants (i) et (ii) sont sous forme de poudre sèche.

6. Médicament selon l'une quelconque des revendications précédentes, **caractérisé en ce que** le rapport du composant (i) sur le composant (ii) est dans la gamme de 1:4 à 1:70.

7. Médicament selon l'une quelconque des revendications précédentes, **caractérisé en ce qu'**il comprend le composant (i) et (ii) sous forme de dose unitaire.

8. Médicament selon l'une quelconque des revendications précédentes, **caractérisé en ce que** les composants (i) et (ii) sont destinés à l'administration par inhalation dans le traitement d'un trouble respiratoire.

9. Médicament selon l'une quelconque des revendications 1 à 8, **caractérisé en ce que** les composants (i) et (ii) sont destinés à l'administration par inhalation dans le traitement de l'asthme.

10. Médicament selon l'une quelconque des revendications précédentes, **caractérisé en ce que** l'administration se fait par inhalation à partir d'un inhalateur de poudre sèche.

11. Médicament selon l'une quelconque des revendications 1 à 9, **caractérisé en ce que** l'administration se fait par inhalation à partir d'un inhalateur sous pression de dose déterminée.

12. Médicament selon l'une quelconque des revendications 1 à 9, **caractérisé en ce que** l'administration se fait par inhalation à partir d'un nébulisateur.

13. Composition pharmaceutique destinée à l'administration par inhalation, **caractérisée en ce qu'**elle comprend conjointement
(i) le formotérol ou un sel physiologiquement acceptable de celui-ci, ou un solvate du sel, ou un solvate de formotérol ; et
(ii) le budésonide.

14. Composition selon la revendication 13, **caractérisée en ce qu'**elle comprend, à titre de composant (i), le fumarate de formotérol dihydraté.

15. Composition selon la revendication 13 ou 14, **caractérisée en ce qu'**elle comprend un diluant ou un support non toxique.

16. Composition selon la revendication 15, **caractérisée en ce que** le diluant ou le support non toxique est le lactose.

17. Composition selon l'une quelconque des revendications 13 à 16, **caractérisée en ce que** le rapport du composant (i) sur le composant (ii) est dans la gamme de 1:4 à 1:70.

18. Composition selon l'une quelconque des revendications 13 à 17, sous forme de dose unitaire.

19. Composition selon l'une quelconque des revendications 13 à 18, **caractérisée en ce que** les composants (i) et (ii) sont chacun sous forme micronisée.

20. Composition selon la revendication 19, **caractérisée en ce qu'**elle est sous forme agglomérée.

21. Inhalateur de poudre sèche, **caractérisé en ce qu'**il contient une composition selon la revendication 20.

22. Capsule, cartouche ou plaquette pour un inhalateur de poudre sèche, la capsule, la cartouche ou la plaquette contenant une composition selon la revendication 20.

23. Composition selon l'une quelconque des revendications 13 à 20, destinée à être utilisée dans le traitement par inhalation de trouble respiratoire.

24. Composition selon l'une quelconque des revendications 13 à 20, destinée à être utilisée dans le traitement par inhalation de l'asthme.

25. Utilisation de
(i) le formotérol ou un sel physiologiquement acceptable de celui-ci, ou un solvate dudit sel, ou un solvate de formotérol ; et
(ii) le budésonide,
dans la fabrication d'une préparation combinée destinée à l'administration par inhalation dans le traitement de trouble respiratoire.

26. Utilisation selon la revendication 25, **caractérisée en ce que** le composant (i) est le fumarate de formotérol dihydraté.

27. Utilisation selon la revendication 25 ou 26, **caractérisée en ce que** le rapport du composant (i) sur le composant (ii) est dans la gamme de 1:4 à 1:70.

28. Utilisation selon l'une quelconque des revendications 25 à 27, **caractérisée en ce que** le régime de dose est de deux fois par jour et la dose du composant (i) est de 6 à 100 µg par jour et la dose du composant (ii) est de 50 à 4 800 µg par jour.

29. Utilisation selon la revendication 28, **caractérisée en ce que** la dose du composant (i) est de 6 à 48 µg par jour et la dose du composant (ii) est de 100 à 1 600 µg par jour.

30. Utilisation de formotérol, ou d'un sel physiologiquement acceptable de celui-ci, ou d'un solvate dudit sel, ou d'un solvate de formotérol, dans la fabrication d'un médicament tel que défini à la revendication 1, pour l'administration par inhalation dans le traitement d'un trouble respiratoire.

31. Utilisation de budésonide dans la fabrication d'un médicament tel que défini à la revendication 1, destiné à l'administration par inhalation dans le traitement de trouble respiratoire.
